# EUROPEAN PATENT APPLICATION

(11) **EP 2 289 937 A1**
(43) Date of publication of application: **02.03.2011**
(21) Application number: 09168530.5
(22) Date of filing: 24.08.2009
(51) Int. Cl.: C07K 16/00

(54) **Production of monoclonal antibodies in vitro**

(71) Applicant: Stücker, Wilfried, 50937 Köln (DE); Jansen, Gabriele, 48153 Münster (DE)
(72) Inventor: Stücker, Wilfried, 50937 Köln (DE); Lüke, Wolfgang, 48153 Münster (DE)
(74) Representative: Habermann, Gert

(57) **Abstract**

The present invention relates to an *in vitro* method for the production of monoclonal antibodies, in particular of IgG type, making use of antigen presentation by dendritic cells.

## Description

The present invention relates to an *in vitro* method for the production of monoclonal antibodies, in particular of IgG type, making use of antigen presentation by dendritic cells.

Thus, this invention describes a new approach for the generation of diagnostic and therapeutic antibodies against animal and human pathogens, bacteria, mycoplasma, virus particles, viruses and their exotoxins, endotoxins, and enterotoxins, including their modifications occurring genetically and by biochemical, chemical and physical processes, as well as for use against therapeutically relevant proteins and tumoral agents, including their modifications occurring genetically and by biochemical, chemical and physical processes. The approach is based on an *in-vitro* procedure that makes it possible to manufacture human monoclonal antibodies using the authentic antigen. In this manner a platform technology is provided that enables the manufacture of monoclonal antibodies of human origin against a multitude of animal and human pathogens, bacteria, mycoplasma, virus particles, viruses and their exotoxins, endotoxins, and enterotoxins, including their modifications occurring genetically and by biochemical, chemical and physical processes, as well as for use against therapeutically relevant proteins and tumoral agents, including their modifications occurring genetically and by biochemical, chemical and physical processes and thereby dispenses with the classical, animal-intensive immunization model and hybridoma technology scheme.

### Background of the invention

### Pathogenic Substances:

Many pathogens, whether they are bacteria, viruses, mycoplasma or other disease-causing toxins, agents or substances, have a direct effect on the survivability of the affected host. A direct immunization is not possible on account of the high toxicity of many pathogenic substances. Instead many toxoids have been employed whose toxicity is generally greatly reduced with aldehyde treatment. These detoxification reactions are effectively not controllable so that immunization occurs with surface epitopes on a relatively random basis. Similar animal-intensive tests aimed at the production of monoclonal as well as polyclonal antibodies against toxins have been reported several times. But despite the considerable effort, they were able to produce only a small quantity of antibodies or sera with an acceptable affinity to the native toxin. The antibodies displayed instead in most cases a much stronger affinity with the toxoid or reacted randomly with other aldehyde protein adducts. As a result, in diagnostic test systems their use yielded only modest sensitivities and specificities. In order to improve these results, expensive and additional affinity purifications and extreme signal strengthening systems like polyHRP had to be employed. (Gessler et al., 2005). An alternative approach, which resulted in antibodies with good performance, was demonstrated by Attree and colleagues (2007). In this approach, recombinant partial toxins were employed for immunization of mice. The performance in the ELISA system of the antibodies generated thereby was relatively good. This technology, however, is not suited for routine use in particular with respect to the emergence of new kinds of toxins or toxin variations due to the time-consuming and difficult process. Therefore the establishment of a highly flexible system for generation of diagnostic and therapeutic antibodies for human application is of major significance.

### Dendritic Cells:

In modern medicine the use of diagnostic and therapeutic antibodies has come to occupy a central role. In particular therapeutic antibodies have found a place in clinical applications. Especially cancer but increasingly also neurodegenerative diseases and allergies are being treated with monoclonal antibodies (Nicolaides et al., 2006). The monoclonal antibodies used in current applications are generally chimeric (mouse/human) antibodies (human proportion 70%) or humanized antibodies (human proportion 95%). In initial human therapeutic trials with the original, non-humanized monoclonal antibodies of nearly 20 years ago it was shown their repeated application led to a strong immune response that significantly limited or destroyed their therapeutic efficacy (Mascelli et al., 2007). To obtain a maximum of safety and and toleration, and in order to eliminate the remainder immunogenetic risk of chimeric or humanized antibodies, other procedures have since been developed which ensure the manufacture of entirely human monoclonal antibodies. These are transgenic mouse systems with an entire human antibody repertoire on the one hand and on the other, human phagocyte libraries with an entire array of human genetic immunoglobulin B-cells. However, all of these methods for the manufacture of chimeric, humanized or human antibodies are very lengthy and costly in their implementation. The immunization of laboratory animals is still required (with the exception of phagocyte libraries) and relatively large quantities of antigens have to be provided to accomplish this. The resulting of necessity heterogeneous expression systems for the recombinant production provide very often antibodies that do not always reflect the authentic conformation and post-translational modifications (Konthur et al., 2005).

An ideal approach for the production of a human monoclonal antibody would circumvent these laborious methods and procedures. This would involve the formation of an authentic antibody by the induction of a natural immune response directly through human blood cells, which could be implemented by an *in vitro* immunization procedure of human peripheral blood lymphocytes (peripheral blood mononuclear cells or PBMCs). First attempts towards such an *in vitro* immunization procedure were developed in the 1980s and 1990s. These immunization procedures were based on the isolation of PMBC, the treatment of PMBCs with L-leucyl-L-leucine methyl ester (LLME) to remove natual killer cells (NK cells) and cytotoxic T cells, which suppress the antigen specific B cell activation, followed by *in vitro* immunization via the addition of the pretreated PBMC and immortalization with the Epstein-Barr-Virus (EBV; Borrebaeck, 1989). With this method a series of highly effective antibodies, in particular neutralizing antibodies against the immunity deficiency virus of Type 1 (HIV-1), were produced (Chin et al., 1995). However, it turned out that no adequate class change (isotope switch) from IgM to IgG type Immunoglobulin was achievable with this *in vitro* immunization procedure. The antibodies produced using this method were overwhelmingly IgM type (Zafiropoulos et al., 1997). Only in recent years has this *in vitro* immunization procedure been improved through the focused use of IL-2, IL-4 und IL-10 cytokines. The use of these cytokines led to the selective induction of Th2 T cells, followed by a marked increase in B cell differentiation towards antibody producing plasma cells (Xu et al., 2004). However, antibodies of the IgM type were still preferentially produced. Parallel to this in vitro immunization procedure of PBMCs, a method for the direct immunization of isolated B cells was described recently (Li et al., 2006). Here, too, only increased IgM production was observed.

All of these efforts towards the development of an efficient *in vitro* immunization procedure indicate that the induced *in vitro* immune reaction involves a B cell response that is independent from T cells and which is characterized by an inadequate B cell activation, expressed in a strong preference for IgM production and an inefficient class change (isotope switch)

It is now generally accepted that the efficient stimulation of naive B cells and their differentiation into antibody producing plasma cells with an efficient class change (isotope switch) is dependent on the following factors:
1. Initially a bonding of an antigen with the B cell receptor must occur.
2. The antigen must then subsequently be internalized with CpG, the ligand for TLR 9 (toll like receptor 9) and then be processed and presented via class II MHC (Ruprecht and Lanzavecchia, 2006).
3. Subsequent to this an efficient helper T cell activation must occur. In this connection a Th2 T cell stimulated with the same antigen must bind through its T cell receptor to the class II MHC presented antigen on the B cell. This bonding is strengthened significantly by CD40/CD40L interaction.
4. Following this the differentiation into plasma cells and their proliferation by cytokine IL-21 can be strengthened significantly (Ettinger et al., 2005).

An essential prerequisite for the efficient generation of antigen stimulated Th2 T cells is the bonding of naive T cells to dendritic cells, which the processed antigen presents via class II MHC molecules. This identifies the dendritic cells as the primary antigen presenting cells which play a central role in the induction of the adaptive immune response (Steinman and Hemmi, 2006). In doing so immature dendritic cells take in antigens through defined receptors, direct these antigens for processing in the exogenous or endogenous processing mode and differentiate in the course of processing into mature dendritic cells. While exogenous processing leads normally to antigen presentation via class II MHC molecules, endogenous processing results in antigen presentation via class I MHC.. Class II MHC presentation leads normally to induction of the humoral immune response and the production of antibodies, while class I MHC presentation presentation activates Th1 T cells, which leads subsequently to induction of the cellular immune response in the form of cytotoxic T cells.

The technical problem underlying the present invention is to provide an improved in *vitro* method for the production of monoclonal antibodies, preferentially of IgG type.

### Summary of the invention

The solution to the above technical problem is characterized by the embodiments of the present invention as defined in the claims.

In particular, the present invention provides a method for the production of monoclonal antibodies, in particular of IgG type, against an antigen *in vitro* comprising the steps of:
(a) preparing dendritic cells from, preferably LLME-treated, peripheral blood mononuclear cells (PBMC)
(b) loading the dendritic cells with said antigen under conditions such that processing of said antigen in the dendritic cells and presentation of the processed antigen on the dendritic cells occurs;
(c) culturing B cells and CD4+ T cells in the presence of the dendritic cells obtained in step (b) such that antigen presentation by the dendritic cells in the presence of the CD4+ T cells to the B cells leads to activation of the B cells to produce antibodies against the antigen; and
(d) isolating one or more clones from the antibody-producing B cells obtained in step (c).

Preferably, the above method further comprises the step (e) of immortalizing the one or more antibody-producing B cells clone(s) obtained in step (d). The immortalization may be carried out using Epstein-Barr-Virus (see Traggai et al., 2004), or may be accomplished by fusion of the clone(s) with myeloma cells (e.g. human myeloma cells, human immortalized primary myeloma cells, human-mouse hetero-myeloma cells, permanent human myeloma cells etc.).

If needed, the above step (b) comprises loading the dendritic cells with an antigen/polymer complex.

According to a preferred embodiment of the invention step (b) is carried out in the presence of one or more Toll-like Receptor (TLR) agonists.

Step (c) of the present invention may be carried out either by
(c1) culturing firstly the dendritic cells obtained in step (b) with CD4+ T cells in order to generate CD4+ T cells of type Th2 and then culturing secondly the obtained CD4+ T cells of type Th2 with B cells; or by
(c2) culturing the dendritic cells obtained in step (b) with CD4+ T cells and B cells simultaneously.

It is to be understood that the term "antigen" according to the present invention means any eptitope-containing object against which antigens can naturally be produced in an organism, in particular humans. Thus the "antigen" may be selected from (human) pathogens, allergens, bacteria, toxins (such as endotoxin, enterotoxins etc.), mycoplasma, fungi and viruses as well as any parts of such antigens.

Preferably, the B cells and/or the CD4+ T cells are isolated from, preferably LLME-treated, PBMCs.

The inclusion of dendritic cells in the development of an efficient *in vitro* immunization procedure according to the present invention for the generation of functional, human monoclonal antibodies is indispensable but has been neglected in investigations undertaken to date. In doing so the essential prerequisite for antibody production must logically be met: the relevant antigens must be captured by the endogenous processing of the dendritic cells and thereby ensure the attainment of an optimal and efficient class II MHC antigen presentation. Should an "escape" of the natural antigen occur from the endosomal compartment into the cytosol, which would lead to the result of an undesired cross presentation of the antigen by way of class I MHC, the antigen must be "forced" into the exogenous processing modus by means of speficic surface modifications. This is feasible through the use of directed "retargeting" strategies with loaded polymers, which can be coupled with ligands for specific cellular receptors in order to ensure a spcecific uptake in endosomes. To support the processing and differentiation of dendritic cells, agonists for endosomal TLR (CpG, RNA) can be co-applied.

The *in vitro* generation of dendritic cells presented for a long time a great challenge. Attempts at the direct isolation of dendritic cells from blood donors did not prove efficient in the past. This is basically because the results were meager. This is probably also the reason why in all previous *in vitro* immunization attempts merely a B cell response independent of T cells was observed because an efficient antigen presentation on account of the lack of dendritic cells was not possible and as a result no activation of Th2 T cells occurred and therefore no helper function was available for the B cells. Only the development of technologies that enabled the generation of dendritic cells from defined stem cell precursors led to a fundamental breakthrough. Monocytes were identified as the principal stem cell precursors, which differentiated into immature DCs after the administration of GM-CSF and IL-4, which in turn are capable of efficient antigen intake (Peters et al., 1993). Further it was shown in recent years that the application of IL-15 instead of IL-4 effectuated the differentiation of the monocytes into immature dendritic cells that showed characteristics of Langerhans cells (Mohamadzadeh et al., 2001).

With the use of these dendritic cells derived from monocytes, with which an efficient antigen presentation and an equally efficient T cell activation can be assured, it is now possible to create a functioning *in vitro* immunization procedure for the generation of human monoclonal antibodies.

The immortalization of human B cells, while possible from a technical standpoint, occurs frequently with less efficiency and loss of antigen specificity on account of the lengthy cultivation process. Using the above-described process - whereby the B cells are expressed with antigens independently of the B cells immortalized with EBV - a fusion occurs with humanized and human-immortalized primary myeloma cells, mixed human-mouse cells or via permanent human myeloma cells including those that have been modified through physical, biochemical or genetic engineered processes, The immortalization of the primary myeloma cells can be achieved in this connection using natural, biochemical, genetic engineering or physical processes. It is important that the fused cell lines can express and secrete typical and functional monoclonal antibodies.

The cultivation of individual antibodies secreting cell clones occurs using specific, generally recognized cell cultivation procedures as well as optimized procedures used for commercial and industrial applications. The harvesting of antibodies occurs using the current state of the art, optimized laboratory techniques.

### Detailed description of the invention

The invention is based on a novel technology that is based on dendritic cells and with which *in vitro* monoclonal antibodies (also human) for use against any antigen including animal and human pathogens, bacteria, mycoplasma, virus particles, viruses and their exotoxins, endotoxins, and enterotoxins, including their modifications occurring genetically and by biochemical, chemical and physical processes, as well as for use against therapeutically relevant proteins and tumoral agents, including their modifications occurring genetically and by biochemical, chemical and physical processes, can be produced.

The procedure offers several advantages:
(i) to produce the antibodies, no immunization of laboratory animals are necessary and only small amounts of the antigen are required;
(ii) the antigen (e.g. a toxin) can be used in its native form;
(iii) toxoidation is thereby dispensed with. In the case of immunization with a toxin, very often antibodies are generated which have either no reactivity or only weak reactivity with the native antigen;
(iv) since the antibodies can be manufactured using human cells, these need not be further humanized for therapeutic purposes and can be applied directly in humans;
(v) the entire procedure offers the possibility to be adapted relatively easily to GMP standards.

### The pathogenic substance:

The chosen animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes, will be cultivated under its natural conditions of growth. By means of an appropriate fermentation small quantities will be produced and purified by way of affinity chromatography. For this purpose commercially available monclonal antibodies will be used that are either anti-toxoid sera with cross-reactivity to the toxin or are aimed against recombinant subunits of the relevant animal and human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes. The relevant animal and human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes will be identified with an indirect ELISA, a competitive ELISA and a sandwich ELISA. In doing so resort will be made to commercially available antibodies.

In vitro Immunization of human PBMC:

*In vitro* immunization is a procedure that was first described about 20 years ago (Borrebaeck, 1989) and that is based on the incubation of isolated human PMBCs with peptides that are derived from different proteins. As a rule this method delivers antigen specific IgM within two to three months, but an efficient "isootope switch"to IgG has not been observed under this method (Xu et al., 2004). It is suggested to use this method for in vitro immunization as a positive control measure. For this purpose donor PBMCs will be treated with LLME (L-Leucyl-L-Leucin Methylester) to remove monocytes, cytotoxic T cells, CD8+ suppresor T cells and NK cells which, as shown in earlier studies, markedly inhibit antibody production (Aiba et al., 2006). The LLME-treated PBMCs are then incubated with the corresponding peptide or antigen in the presence of different cytokines (IL-2, IL-4, IL-10). Then the in vitro immunized B cells are immortalized with Epstein-Barr-Virus (EBV) and cloned biologically in order to find high-producing B cell clones.

### Generation of dendritic cells:

Attempts to isolate DCs directly from donor or patient blood samples have proven inefficient in the past. This was essentially due to the fact that the quantity obtained was small. Only with the development of technologies that permit the generation of DCs from defined precursor blood cells did a basic breakthrough occur. Monocytes that differentiate into immature DCs after administration of GM-CSF and IL-4 were identified as the basic precursor cells which are capable of efficient antigen processing (Peters et al., 1993). Furthermore it has been shown in recent years that the administration of IL-15 in place of IL-4 caused differentiation of the monocytes into immature dendritic cells that showed the characteristics of Langerhans cells (Mohamadzadeh et al., 2001). Also, these dendritic cells proved to be potent antigen-presenting cells.

Three strategies may be pursued for the generation of monocyte-derived dendritic cells (MDDC). The results will show which method delivers the dendritic cell population with the highest uniformity along with simultaneous expression of the surface markers that are typical for immature dendritic cells.

### Antigen uptake and processing:

This point takes a central role because how antigens are taken up in dendritic cells and how their subsequent processing and presentation occur are of decisive importance for the resulting immune response. For animal and human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes no results are known yet. Therefore they must be taken up in intracellular, endosomal compartments that result in the targeted proteolytic processing of the relevant proteins and thereby produce via this exogenous processing method an efficient antigen presentation by way of class II MHC molecules, which is a basic requirement for the later *in vitro* generation of antibodies.

If the loading of MDDCs with soluble monomers of animal and human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes monomers do not lead to an efficient class II MHC presentation, care must be taken by specific "retargeting" strategies with the aid of loaded polykations, which can be coupled with ligands for specific cellular receptors, to ensure that animal and human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes, is funneled into the exogenous processing mode (Lüke et al., 2003).

### Influencing maturation and antigen presentation:

The efficient maturation and antigen presentation of dendritic cells depends on the efficient induction and secretion of class I interferon (IFN-α/β), as shown by the example of viruses. This function can be strengthened substantially by binding of virus-specific ligands to particular TLRs. For viruses that succeed in entering cells through endocytosis, TLRs 7/8 and 9 appear to play an important role in this respect. Apparently these TLRs bind in the endosomes to the released viral genomes.

Thereby TLRs 7/8 appear to be responsible for binding to single-stranded RNA genomes, while TLR 8 binds to unmethylated CpG motifs in viral DNA genomes (Kawai and Akira, 2006).

Almost nothing is known about the role of TLRs in the maturation and antigen presentation of dendritic cells after uptake of soluble, monomer proteins. Therefore the viral situation will be followed by coapplication of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes with ligands for TLRs 7/8 (ssRNA) and 9 (CpG-ODN). The nature and manner of coapplication is of decisive importance.

### Polarization of naive CD4⁺ T cells:

The generation of Th2 type CD4⁺ helper T cells is of prime significance for the induction of an adaptive humoral immune response. An essential requirement for this is the class II MHC antigen presentation by dendritic cells as well as the regulation of the costimulating surface molecules CD40, CD80, CD83, CD86 and OX40, typical for matured dendritic cells. Moreover, the cytokine milieu is of great importance. After binding of naive CD4⁺ T cells to the antigen presenting class II molecules on the dendritic cells through the T cell receptor, the polarization of tghe bonded T cells to Th1 or Th2 cells is initiated. In the polarization into Th2 cells, costimulation through the interaction of CD80/CD86 and CD80L/CD86L (CD28) is of great importance and is strengthened by interaction with OX40/OX40L. In addition, the ratio of dendritic cells to naive CD4⁺ T cells and the antigen density on the dendritic cells appear to be important for the polarization into Th2 cells (Tanaka et al., 2000). A ratio of 1:300 and a low antigen density promotes Th2 generation. Cytokines IL-4, 5, 6 and 10 encourage Th2 polarization. This scenario should demonstrate the use of *in vitro* methods for the generation of Th2 cells.

### Acitvation of naive B cells:

Previous studies have demonstrated that B cells can be stimulated *in vitro* and as a consequence secrete antibodies. The secreted antibodies were above all of IgM type, however, and an efficient "isotype switch" (class change) to the IgG type could not be observed. But this research was done essentially on "memory" B cells, whereas the stimulability of naive B cells has been studied only recently. Such studies have shown that three signals are necessary for effective activation: stimulation of the B cell receptor, the costimulation via CD40/CD40L by antigen-specific Th2 helper cells and interaction with TLR7/9 Agonists. However, these studies were carried out almost entirely under artificial conditions. For example the B cell rfeceptor was not stimulated with an authentic antigen and the T helper cells similarly were not activated by authentic antigen. Therefore, the optimal conditions for the activation of naive B cells with authentic antigen must be determined.

### The figures show:

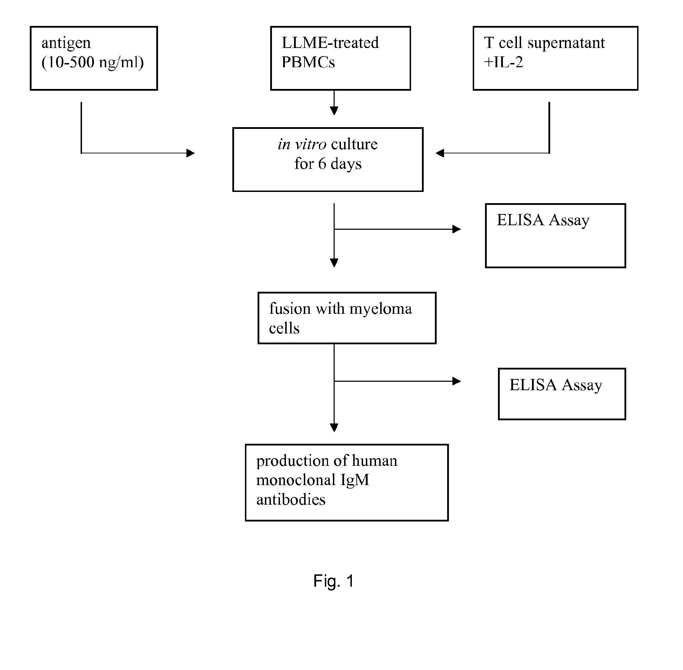

- Fig. 1: shows a schematic representation of an *in vitro* method for the production of monoclonal antibodies according to the prior art (see Borrebaeck, 1989).

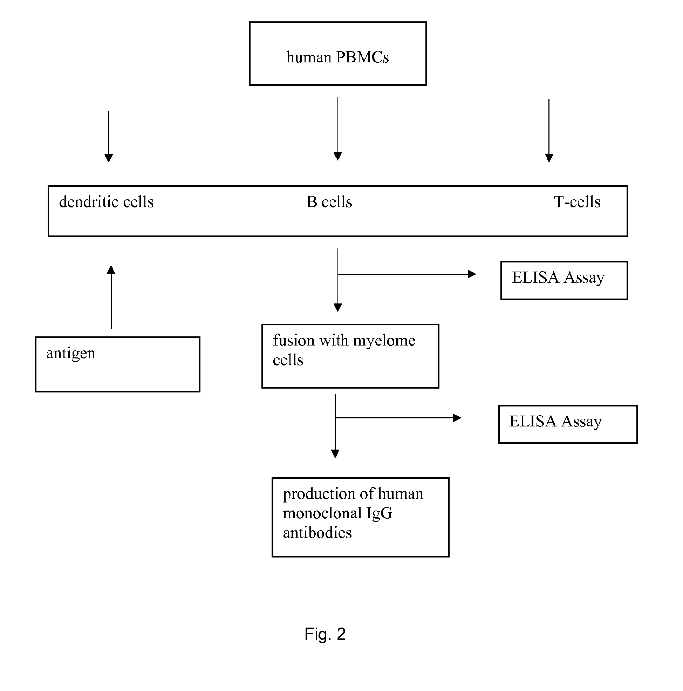

- Fig. 2: shows a schematic representation of an *in vitro* method for producing monoclonal antibodies according to the present invention.

### Best mode for carrying out the invention

The present invention is further illustrated by the following non-limiting examples:

### EXAMPLES

### Example 1: Generation of sufficient quantities of peripheral lymphocytes:

The starting cell population from which the dendritic cells and the cells that serve to generate the B cell clones are produced, are peripheral blood mononuclear cells (PBMC). These are produced from "buffy coats" or from fresh blood of human donors by means of density gradient centrifugation using lymphoprep. After homologation of the expression status of the surface markers CD3, CD4, CD8, CD14, CD19, CD20, CD25, CD26 and CD34, the PBMCs are cryopreserved with the number of cells at 10' in the presence of of DMSO and as autologous serum cryoconserved and stored on liquid N₂.

### Example 2: Purification and characterization of the lymphocyte subpopulations:

***CD4*⁺*T cells:*** Here particularly the CD4⁺ T cells are of special importance. Therefore the CD4⁺ T cells are isolated from the aggregate population of PMBCs using appropriate compartmentation kits made by the Miltenyi company. The cell population thereby purified is then examined as to its immunological status. For the CD4⁺ T cells the Th1/Th2 proportion is particularly important and whether "memory" CD4⁺ T cells are existent. This is to be examined by means of proliferation tests using a recognized method (Voss et al., 1992; 1993; Dittmer et al., 1994; Weber et al., 2001). As stimulating antigens, tetanus toxoid (TT), Phytahaemagglutinin (PHA) and a recombinant form of the human Polyomavirus JCV among others will be used. The cytokine pattern obtained will provide information about the composition of the CD4⁺ T cell population.

***B cells:*** The B cells are similarly isolated from the aggregate population of the PBMCs using apppropriate compartmentation kits made by the Miltenyi company. As with the CD4⁺ T cells, the B cell population is examined as to the ratio of "naive" B cells to "memory" B cells. This is done by the analysis of defined surface markers using flow cytometry. The expression pattern of the surface markers will give information on the ratio of "naive" B cells to "memory" B cells. Parallel to this the serological status of the donors will be ascertained.

Further studies will be done using the PBMCs only of those donors having a minimal and defined immune status.

### Example 3: dendritic cells:

### A. Generation:

### Method 1:

1. Enrichment of monocytes by way of plastic adherence.
2. Genera of DCs with GM-CSF and IL-4 or IL-15 in DC-Medium (CellGenix) with variation of cell numbers, times and cytokine concentrations.
3. Characterization of immature DCs by way of FACS analysis.

### Method 2:

1. Indirect isolation of monocytes (Monocyte Isolation Kit II, Miltenyi).
2. Further enrichment with plastic adherence.
3. Generation and characterization of DCs as described under method 1.

### Method 3:

1. Positive selection of Monocytes (CD14 MicroBeads, Miltenyi).
2. Generation of DCs in suspension in DC Bags (CellGenix) with variation of cell numbers, times and cytokine concentrations.
3. Characterization of DCs as described under method 1.

### B. Loading and Maturation:

1. Loading of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes with the corresponding polymers.
2. Loading of MDDCs withanimal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes or the corresponding pathogen/tumor antigen polymer complex in varying concentrations.
3. Examination of the differentiation of the loaded MDDCs with and without addition of signaling components for maturation induction per the FACS analysis using differing periods of time.
4. Determination of IFN-α/β/y as well as IL-6, IL-10 and IL-12 secretion using different periods of time.

### C. Intracellular trafficking and processing:

1. Determination of the uptake mechanism by the application of defined uptake inhibitors.
2. Determination of intracellular concentration of animal and human pathogen, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes using different periods of time.
3. Determination of the concentration of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes in varying cellular compartments under subcellular fractionation and using different periods of time.
4. Examination of intracellular trafficking using immunoflourescence and electron microscopy.
5. Examination of the costimulating maturation markers (CD40, CD80, CD83, CD86) using flow cytometry.

As an alternative to the polymer complexes, immune complexes (IC) of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes are used for loading of the MDDCs. For this immune sera are used against the animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes. The loading of the MDDCs and further examination of maturation and trafficking are carried out as described above.

### D. Coapplications of TLR agonists with the assistance of virus like particles (VLP)

1. The nucleic acids are packaged in virus like particles (VLP).
2. The VLPs are loaded with the identical polymers as the coapplied animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes in order to assure uptake into the identical cell compartment.
3. The coaaplication occurs in varying ratios.

Previous studies have shown that such VLP DNA- and RNA molecules package superbly and after appropriate retargeting bring the nucleic acids into the desired compartments of MDDCs with high levels of efficiency (Goldmann et al., 1999; Petry et al., 2003; Lüke et al., 2003; Lüke et al., unpublished observation).

### E. Incubation of dendritic cells with naive CD4+ T cells

The dendritic cells, with different antigen densities, are incubated with the naive CD4⁺T cells in differeing ratios (1:50; 1:100; 1:200,1:400; 1:800).

The secretion of cytokines IL-4, IL-5, IL-6, IL-10, IL-12 and IFN- y is observed over time. In addition the development of the Th2 surface marker CCR2, 3 and 4 is observed. The generation of Th2 T cells is carried out as well in the presence of antibodies against IL-12 and IFN-γ (Th1-promoting cytokines). In addition, research is to be done as to whether the generation of Th2 cells can be improved in the presence of IL-4, IL-6 and IL-10.

### Example 4: Acitvation of naive B cells

For this naive B cells are isolated from PBMCs by negative selection (Miltenyi) in highly purified form. To evaluate the antigen-specific activation, the B cells are incubated with the different forms of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes, together with different TLR 7/9 agonists. The activation is promoted by costimulation with anti-CD40 antibodies, or specific Th2 helper T cells of animal and human pathogen, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes, in different ratios. Moreover, the influence of different cytokines such as IL-2, IL-4, IL-6, IL-10 and IL-21 are examined alone or in combination with respect to activation of the B cells. The ultimate generation of antibody secreting plasma cells is determined at different periods of time by examining the cell culture supernatant for antibody production.

In order to facilitate results from the investigations, the cytidine deaminase (the key enzyme for the "isotype switch") is set using real time PCR. With the assistance of real time PCR, the Xbox binding protein (important for the differentiation into plasma cells) is to be ascertained.

### Example 5: Orchestriation of the individual components:

### A. Antibody secreting plasma cells from PBMCs

### System 1:

1. Generation of the appropriate immature dendritic cells.
2. Admixture of these dendritic cells to the LLME-treated PBMCs with variation of the cell tally.
3. Addition of the apppropriate preparation of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes.
4. Incubation for up to ten days with appraisal of the antibody secretions on each day.

### System 2:

1. Generation of appropriate mature dendritic cells that have been loaded with differing preparations of animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes.
2. Admixture of these dendritic cells to the LLME-treated PBMCs with variation of the cell tally.
3. Incubation for up to ten days.
4. Further treatment as described under system 1.

### System 3:

1. Generation of mature dendritic cells.
2. Incubation isolated naive T cells and generation of Th2 helper T cells.
3. Admixture of the LLME-treated PMBCs.
4. Incubation for up to ten days.
5. Further treatment as described under system 1.

### B. Antibody secreting plasma cells from B cells and Th2 cells

### System 4:

1. Generation of activated B cells.
2. Admixture of the activated B cells to system 2.
3. Incubation for up to ten days.
4. Further treatment as described under system 1.

### System 5:

1. Generation of Th2 helper T cells.
2. Generation of activated B cells.
3. Admixture of the activated B cells to the activated TH2 helper T cells.
4. Incubation for up to ten days.
5. Further treatment as described under system 1.

### Example 6: Generation of B cell clones:

### A. Isolation and immortalization of the B cells

To harvest the B cell clones the activated B cells are isolated from the corresponding system and immortalized with a new method (Traggiai et al., 2004). To this end the isolated B cells are restimulated with the appropriate antigen and corresponding TLR agonists and immportalized with Epstein-Barr-Virus.

### Example 7: Characterization of the antibodies:

### A. Purification of the antibodies

About two weeks after restimulation and immportalization the supernatant from the cultures are examined for antibody production and the positive cultures are cloned biologically and expanded. The secreted antibodies are then purified from the supernatant using affinity chromatography.

### B. Determination of the dissociation constants and the capacity of the antibodies

The dissociation constants of the purified antibodies is determined in competition tests first. Then the diagnostic and neutralizing capacity of the purified antibodies is measured. Different biological tissues and liquids with different concentrations of animal and human pathogen, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes are "spiked" for the diagnostic examination. The antigen identification occurs by means of the purified antibodies in the ELISA.

### C. Neutralization capacity

For the studies on neutralization, the binding inhibition of animal and human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes to various target cells is determined by way of the purified antibodies. For this purpose the chosen animal or human pathogens, bacteria, mycoplasma, virus particles, viruses and their exotoxins, endotoxins, and enterotoxins, including their modifications occurring genetically and by biochemical, chemical and physical processes, as well as therapeutically relevant proteins and tumoral agents, including their modifications occurring genetically and by biochemical, chemical and physical processes are is incubated in the presence of known different antibody concentrations. Identification of the bonded pathogenic agent or the tumoraL antigen follows then using Western Blot.

### References cited:

The disclosure of the following literature is hereby included in the present description in its entirety by reference:
Aiba, Yamashita, Katakura, Furukawa, Matsumoto, Tomimatsu, Teruya and Shirahata: Identification of genes involved in the suppression of antibody production from human peripheral blood lymphocytes. Biosci. Biotechnol. Biochem. 70: 966-970 (2006)
Borrebaeck: Strategy for the production of human monoclonal antibodies using in vitro activated B cells. J. lmmunol. Meth. 123: 157-165 (1989)
Chin, Malmborg, Kristensson, Hinkula, Wahren and Borrebaeck: Mimicking the humoral immune response in vitro results in antigen-specific isotype switching supported by specific autologous T helper cells: generation of human HIV-1-neutralizing IgG monoclonal antibodies from naïve donors. Eur. J. Immunol. 25: 657-663 (1995)
Ettinger, Sims, Fairhurst, Robbins, daSilva, Spolski, Leonard and Lipsky: IL-21 induces differentiation of human naïve and memory B cells into antibody-secreting plasma cells. J. Immunol. 176: 7867-7879 (2005)
Fillatreau and Manz: Tolls for B cells. Eur. J. Immunol. 36: 798-801 (2006)
Gessler, Hampe and Böhnel: Sensitive detection of botulinum neurotoxin types C and D with an immunoaffinity chromatographic column test. App. Environ. Microbiol. 71: 7897-7903 (2005)
Kawai and Akira: Innate immune recognition of viral infection. Nat. Immunol. 7: 131-137 (2006)
Konthur, Hust and Dübel: Perspectives for systematic in vitro antibody generation. Gene 364: 19-29 (2005)
Li, Sai, Berger, Chao, Davidson, Deshmukh, Drozdowski et al.: Human antibodies for immunotherapy development generated via a human B cell hybridoma technology. Proc. Natl. Acad. Sci. USA 103: 3557-3562 (2006)
Maselli, Zhou, Sweet, Getsy, Davis, Graham and Abernethy: Molecular, Biologic and Pharmacokinetic properties of monoclonal antibodies: Impact of these parameters on early clinical development. J. Clin. Pharmacol. OnlineFirst March 22 (2007)
Mohamadzadeh, Berard, Essert, Chalouni, Pulendran, Davoust, Bridges, Palucka and Banchereau: Interleukin 15 skews monocyte differentiation into dendritic cells with features of Langerhans Cells. J. Exp. Med. 194: 1013-1019 (2001).
Nicolaides, Sass and Grasso: Monoclonal antibodies: A morphing landscape for therapeutics. Drug Develop. Res. 67: 781-789 (2006)
Peters, Xu, Ruppert, Ostermeier, Friedrichs and Giseler: Signals required for differentiating dendritic cells from human monocytes in-vitro. Advan. Exp. Med. Biol. 329: 275-280 (1993).
Ruprecht and Lanzavecchia: Toll-like receptor stimulation as a third signal required for activation of human naive B cells. Eur. J. lmmunol. 36: 810-816 (2006).
Steinman and Hemmi: Dendritic Cells: Translating innate to adaptive immunity. CTMI 311: 17-58 (2006)
Tanaka, Demeure, Rubio, Delespesse and Sarfati: Human monocyte-derived dendritic cells induce naive T cell differentiation into T helper Cell type 2 (Th2) or Th1/Th2 effectors: Role of stimulator/Responder Ratio. J. Exp. Med. 192: 405-411 (2000)
Traggiai, Becker, Subbarao, Kolesnikova, Uematsu, Gismondo, Murphy, Rappuoli and Lanzavecchia: An efficient method to make human monoclonal antibodies from memory B cells: potent neutralization of SARS coronavirus. Nat Med 10: 871-875 (2004)
Zafiropoulos, Andersson, Krambovitis and Borrebaeck: Induction of antigen-specific isotype switching by in vitro immunization of human naïve B lymphocytes. J. Immunol. Meth. 200: 181-190 (1997)
Xu, Katakura, Yamashita, Fang, Tamura, Matsumoto et al.: IL-10 augments antibody production in in vitro immunized lymphocytes by inducing a Th2-type response and B cell maturation. Biosci. Biotechnol. Biochem. 68: 2279-2284 (2004)
Attree, Gros and Miethe: A sensitive and rapid immuno-filtration assay for botulinum-toxin A and its comparison with ELISA. Proceedings of 6th International Conference Munich (2004)
Dittmer, Luke, Stahl-Hennig, Hunsmann and Voss: Virus-specific helper T cell response of macaques immunized with inactivated whole SIV is not correlated with reactivity against cellular components of vaccine preparation. AIDS Res. Hum. Retroviruses 10: 329-330 (1994)
Goldmann, Petry, Frye, Ast, Ebitsch, Jentsch, Kaup, Weber, Trebst, Nisslein, Hunsmann, Weber and Luke: Molecular cloning and expression of major structural protein VP1 of the human polyomavirus JC virus: Formation of virus-like particles usefull for immunological and therapeutic studies. J. Virol. 73: 4465-4469 (1999)
Luke, Petry, Ast, Wilke, Goldmann, Wagner and Schnabelrauch: Composition for transferring active compounds in a cell-specific manner. DE10131145A1 (filed 2001), EP1270586B1 (date of patent 2006), JP2003061693 (filed 2003), US2003044961A1 (filed 2002)
Petry, Goldmann, Ast and Luke: The use of virus-like particles for gene transfer. Curr. Opin. Mol. Ther. 5: 524-528 (2003)
Voss, Nick, Stahl-Hennig, Coulibaly, Petry, Luke and Hunsmann: Potential significance of the cellular immune response against the macaque strain of simian immunodeficiency virus (SIVmac) in immunized and infected rhesus macaques. J. Gen. Virol. 73: 2273-2281 (1992)
Voss, Dittmer, Coulibaly, Makoschey, Petry, Luke and Hunsmann: Differences in the B and T cell immune response to the envelope glycoprotein 130 (gp130) of the macaque strain of simian immunodeficiency virus (SIVmac), induced by immunization of rhesus macaques with virus-derived or vaccinia virus-expressed gp130. J. Gen. Virol. 74: 1757-1763 (1993)
Weber, Goldmann, Kramer, Kaup, Pickhardt, Young, Petry, Weber and Luke: Cellular and humoral immune response in progressive multifocal leukoencephalopathy. Ann Neurol. 49: 636-642 (2001)

## Claims

1. A method for the production of monoclonal antibodies against an antigen *in* vitro comprising the steps of:
(a) preparing dendritic cells from, preferably LLME-treated, peripheral blood mononuclear cells (PBMC)
(b) loading the dendritic cells with said antigen under conditions such that processing of said antigen in the dendritic cells and presentation of the processed antigen on the dendritic cells occurs;
(c) culturing B cells and CD4+ T cells in the presence of the dendritic cells obtained in step (b) such that antigen presentation by the dendritic cells in the presence of the CD4+ T cells to the B cells leads to activation of the B cells to produce antibodies against the antigen; and
(d) isolating one or more clones from the antibody-producing B cells obtained in step (c).

2. The method of claim 1 further comprising the step
(e) immortalizing the one or more antibody-producing B cells clone(s) obtained in step (d).

3. The method of claim 2 wherein the B cell clone(s) is/are immortalized using EBV (Epstein-Barr-Virus).

4. The method of claim 2 wherein the B cell clone(s) is/are immortalized by fusion with myeloma cells.

5. The method of claim 1 wherein step (b) comprises loading the dendritic cells with an antigen/polymer complex.

6. The method of claim 1 wherein step (b) is carried out in the presence of one or more Toll-like Receptor (TLR) agonists.

7. The method of claim 1 wherein step (c) comprises
(c1) culturing firstly the dendritic cells obtained in step (b) with CD4+ T cells in order to generate CD4+ T cells of type Th2 and then culturing secondly the obtained CD4+ T cells of type Th2 with B cells; or
(c2) culturing the dendritic cells obtained in step (b) with CD4+ T cells and B cells simultaneously.

8. The method of claim 1 wherein the antigen is selected from the group consisting of human pathogens, allergens, bacteria, toxins, mycoplasma, fungi and viruses.

9. The method of claim 1 wherein the CD4+ T cells are isolated from, preferably LLME-treated, peripheral blood mononuclear cells.

10. The method of claim 1 wherein the B cells are isolated from, preferably LLME-treated, peripheral blood mononuclear cells.

11. The method of claim 1 wherein the antibodies are of IgG type.
